# FASCICULE DE BREVET EUROPEEN

(11) **EP 2 678 268 B1**
(45) Date de publication et mention de la délivrance du brevet: **19.10.2016**
(21) Numéro de dépôt: 12704838.7
(22) Date de dépôt: 22.02.2012
(51) Int. Cl.: C01B 3/34, C07C 1/04, C25B 15/02, C25B 1/04, C10J 3/00, C07C 29/151

(54) **MÉTHODE DE PRODUCTION DE MÉTHANOL OU D'HYDROCARBURES À PARTIR D'UNE MATIÈRE CARBONÉE, AVEC UNE ÉTAPE DE REFORMAGE DONT LES CONDITIONS DE FONTIONNEMENT SONT AJUSTÉES SÉLECTIVEMENT**
VERFAHREN ZUR HERSTELLUNG VON METHANOL ODER KOHLENWASSERSTOFFEN AUS EINEM KOHLENSTOFFMATERIAL MIT EINEM REFORMIERUNGSSCHRITT UND WAHLWEISE ANGEPASSTEN BETRIEBSBEDINGUNGEN
METHOD FOR PRODUCING METHANOL OR HYDROCARBONS FROM A CARBON MATERIAL, INCLUDING A REFORMING STEP, THE OPERATING CONDITIONS OF WHICH ARE SELECTIVELY ADJUSTED

(30) Priorité: 22.02.2011 FR 1151408
(43) Date de publication de la demande: 01.01.2014
(73) Titulaire: AREVA, 92400 Courbevoie (FR)
(72) Inventeur: LECOMTE, Michel, F-92500 Rueil Malmaison (FR); MOURGUES CODERN, Alejandro Carlos, F-71200 Le Creusot (FR)
(74) Mandataire: Lavoix
(86) Numéro de dépôt international: PCT/EP2012/053014
(87) Numéro de publication internationale: WO 2012/113832

(56) Documents cités:
- WO-A1-2006/099573
- US-A1- 2003 065 042
- US-A1- 2010 175 320
- GALINDO CIFRE ET AL: "Renewable hydrogen utilisation for the production of methanol", ENERGY CONVERSION AND MANAGEMENT, ELSEVIER SCIENCE PUBLISHERS, OXFORD, GB, vol. 48, no. 2, 1 février 2007 (2007-02-01), pages 519-527, XP022109818, ISSN: 0196-8904, DOI: 10.1016/J.ENCONMAN.2006.06.011
- MIGNARD D ET AL: "On the use of electrolytic hydrogen from variable renewable energies for the enhanced conversion of biomass to fuels", CHEMICAL ENGINEERING RESEARCH AND DESIGN, PART A, INSTITUTION OF CHEMICAL ENGINEERS, XX, vol. 86, no. 5, 1 mai 2008 (2008-05-01), pages 473-487, XP022628561, ISSN: 0263-8762, DOI: 10.1016/J.CHERD.2007.12.008 [extrait le 2008-03-04]
- HULTEBERG P C ET AL: "A study of combined biomass gasification and electrolysis for hydrogen production", INTERNATIONAL JOURNAL OF HYDROGEN ENERGY, ELSEVIER SCIENCE PUBLISHERS B.V., BARKING, GB, vol. 34, no. 2, 1 janvier 2009 (2009-01-01), pages 772-782, XP025893328, ISSN: 0360-3199, DOI: 10.1016/J.IJHYDENE.2008.10.073 [extrait le 2008-12-13]

## Description

Selon un premier aspect, l'invention concerne une méthode de production de méthanol ou hydrocarbures à partir d'au moins une matière carbonée.

US 2010/0022669 décrit un procédé de production d'hydrocarbures liquides à partir d'une matière carbonée telle que du méthane ou de la biomasse, en utilisant une source d'énergie renouvelable de puissance variable, une unité de génération d'oxygène (unité de séparation d'air ou électrolyseur) consommant une fraction de l'énergie électrique, un reformeur consommant l'oxygène généré et une autre fraction de l'énergie électrique, et une unité de fabrication d'hydrocarbures liquides à partir du gaz synthétique produit par le reformeur.

Ce document envisage plusieurs solutions pour résoudre le problème de la variabilité de la puissance électrique disponible. Une première solution envisagée est de faire varier la quantité d'oxygène fournie au reformeur, pour rendre celui-ci plus ou moins endothermique ou exothermique et modifier la puissance électrique nécessaire pour le chauffage. D'autres solutions consistent à baisser la capacité de production d'hydrocarbures liquides, à utiliser une source électrique d'appoint pour le chauffage du reformeur, à utiliser une source de chaleur d'appoint ou encore à utiliser un stockage tampon d'oxygène.

Ces procédés sont peu efficaces, et permettent difficilement de compenser une baisse de la puissance électrique disponible importante et de longue durée.

US 2003/0065042 A1 décrit une méthode et unité de production de méthanol à partir d'une matière carbonée.

Dans ce contexte, l'invention vise à proposer une méthode qui soit plus robuste, et qui puisse tolérer des baisses de puissance électrique plus longues et plus prononcées.

A cette fin, l'invention porte sur une méthode de production de méthanol ou d'hydrocarbures à partir d'au moins une matière carbonée, la méthode comprenant les étapes suivantes :
- produire un flux de gaz de synthèse à partir de la matière carbonée, selon un procédé comprenant au moins une opération de reformage d'un flux de gaz intermédiaire issu de la matière carbonée, le flux de gaz de synthèse comprenant au moins de l'hydrogène et du monoxyde de carbone, le gaz de synthèse ayant un premier ratio molaire hydrogène/monoxyde de carbone dans des premières conditions opératoires pour l'opération de reformage ;
- produire un flux d'hydrogène à partir d'une matière première hydrogénée et d'une première puissance électrique consommée, le flux d'hydrogène ayant un premier débit molaire pour ladite première puissance électrique consommée ;
- produire le méthanol ou les hydrocarbures à partir du flux de gaz de synthèse et du flux d'hydrogène ;
- baisser la puissance électrique consommée pour produire le flux d'hydrogène jusqu'à une seconde puissance électrique inférieure à la première puissance électrique, le flux d'hydrogène ayant un second débit molaire inférieur au premier débit molaire pour la seconde puissance électrique consommée, et se placer dans des secondes positions opératoires différentes des premières pour l'opération de reformage pour compenser la baisse du débit molaire du flux d'hydrogène, le gaz de synthèse ayant un second ratio molaire hydrogène/monoxyde de carbone supérieur au premier dans les secondes conditions opératoires.

La méthode peut encore comporter une ou plusieurs des caractéristiques ci-dessous, considérées individuellement ou selon toutes les combinaisons techniquement possibles :
- dans les premières conditions opératoires, l'opération de reformage est effectuée à une première température, dans les secondes conditions opératoires, l'opération de reformage est effectuée à une seconde température inférieure à la première température ;
- dans les premières conditions opératoires, l'opération de reformage est effectuée à une première pression, dans les secondes conditions opératoires, l'opération de reformage est effectuée à une seconde pression inférieure à la première pression ;
- l'opération de reformage est effectuée dans une unité de reformage recevant en entrée une pluralité de flux d'entrée contenant des molécules organiques, dont le flux intermédiaire, l'unité de reformage recevant des différents flux d'entrée au total un débit molaire total d'atomes de carbone contenus dans les molécules organiques et un débit molaire total d'eau H₂O, l'opération de reformage passant des premières conditions opératoires aux secondes conditions opératoires par modification d'un ratio du débit molaire total d'eau H₂O sur le débit molaire total d'atomes de carbone contenus dans les molécules organiques ;
- le ratio du débit molaire total d'eau H₂O sur le débit molaire total d'atomes de carbone contenus dans les molécules organiques est égal à une première valeur dans les premières conditions opératoires, et est égal à une seconde valeur supérieure à la première dans les secondes conditions opératoires ;
- la seconde valeur est comprise entre 1,1 et 5 fois la première valeur, de préférence entre 1,5 et 3 fois la première valeur ;
- l'opération de reformage est effectuée dans une unité de reformage recevant en entrée une pluralité de flux d'entrée, dont le flux intermédiaire, l'unité de reformage recevant des différents flux d'entrée au total un débit molaire total d'atomes de carbone contenus dans les molécules organiques et un débit molaire total d'oxygène O₂, l'opération de reformage passant des premières conditions opératoires aux secondes conditions opératoires par modification d'un ratio du débit molaire total d'oxygène sur le débit molaire total d'atomes de carbone contenus dans le molécules organiques ;
- le flux d'hydrogène est produit à partir d'électricité fournie par un réseau de distribution électrique desservant d'autres consommateurs ; et
- le flux intermédiaire est obtenu par gazéification de la matière carboné.

Selon un second aspect, l'invention porte sur une installation de production de méthanol ou d'hydrocarbures à partir d'au moins une matière carbonée, l'installation comprenant :
- une unité de production d'un flux de gaz de synthèse à partir de la matière carbonée, comprenant au moins une unité de reformage d'un flux de gaz intermédiaire issu de la matière carbonée, le flux de gaz de synthèse comprenant au moins de l'hydrogène et du monoxyde de carbone, le gaz de synthèse ayant un premier ratio molaire hydrogène/monoxyde de carbone dans des premières conditions opératoires pour l'opération de reformage ;
- une unité de production d'un flux d'hydrogène à partir d'une matière première hydrogénée et d'une première puissance électrique consommée, le flux d'hydrogène ayant un premier débit molaire pour ladite première puissance électrique consommée ;
- une unité de production de méthanol ou d'hydrocarbures à partir du flux de gaz de synthèse et du flux d'hydrogène ;
- une unité de pilotage programmée pour baisser la puissance électrique consommée pour produire le flux d'hydrogène jusqu'à une seconde puissance électrique inférieure à la première puissance électrique, le flux d'hydrogène ayant un second débit molaire inférieur au premier débit molaire pour la seconde puissance électrique consommée, l'unité de pilotage étant programmée pour faire passer l'unité de reformage dans des secondes conditions opératoires différentes des premières pour compenser la baisse du débit molaire du flux d'hydrogène, le gaz de synthèse ayant un second ratio molaire hydrogène/monoxyde de carbone supérieur au premier dans les secondes conditions opératoires.

D'autres caractéristiques et avantages de l'invention ressortiront de la description détaillée qui en est donnée ci-dessous, à titre indicatif et nullement limitatif, en référence aux figures annexées, parmi lesquelles :
- la figure 1 est une représentation schématique simplifiée d'une installation de production d'hydrocarbures conforme à un premier mode de réalisation de l'invention ;
- la figure 2 est une représentation schématique similaire à celle de la figure 1, pour une installation de production conforme à un second mode de réalisation de l'invention ;
- les figures 3 à 8 sont des représentations graphiques de l'évolution des fractions molaires en base sèche du CH₄, H₂, CO et CO₂ à l'équilibre et du taux de conversion du méthane à la sortie du reformeur, en fonction de la température de réaction, à 30 bars, pour différentes conditions d'entrée dans le reformeur ;
- les figures 9 et 10 sont des graphiques illustrant l'évolution des rapports CO sur CO₂ et H₂ sur CO à l'équilibre, en fonction de la température de réaction, à 30 bars, pour un reformeur fonctionnant dans les conditions opératoires des figures 3 à 8.

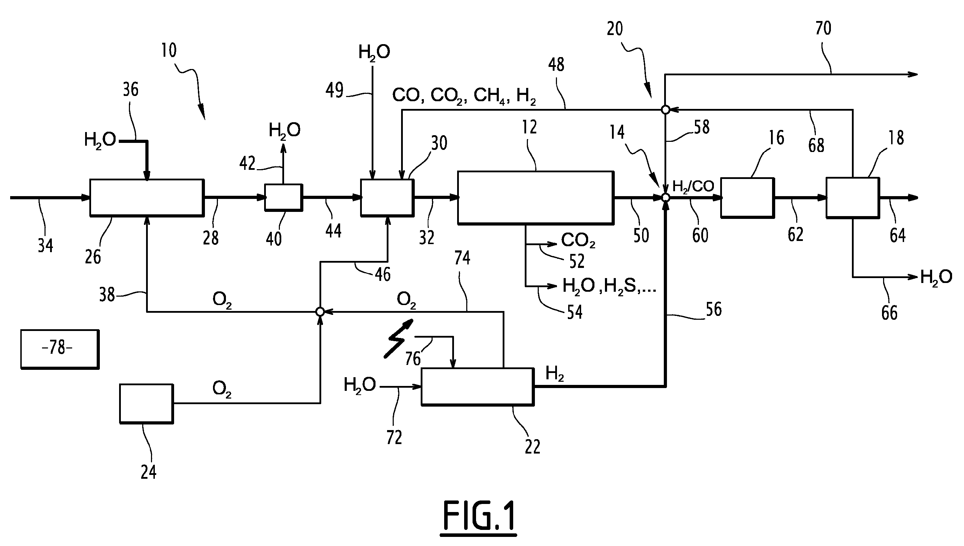

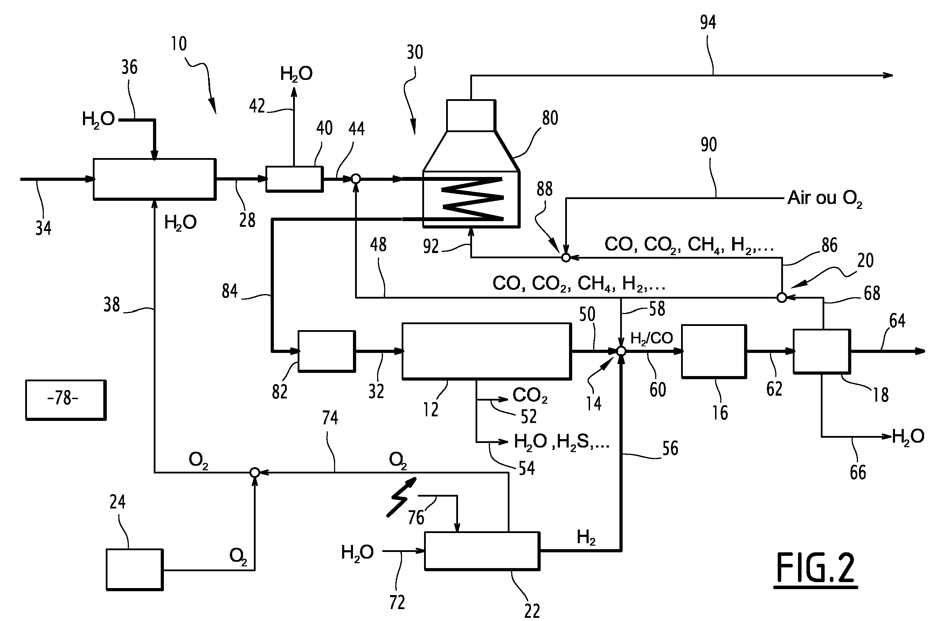

Sur les figures 1 et 2, chaque rectangle correspond à la fois à une étape ou à une sous-étape de la méthode de l'invention, et à l'unité de l'installation industrielle correspondante. L'installation sera décrite comme comprenant des unités, correspondant à des étapes ou sous-étapes du procédé.

Les installations représentées de manière schématique sur les figures 1 et 2 visent à produire du méthanol et ou des hydrocarbures à partir d'une matière carbonée. La matière carbonée peut comprendre un ou plusieurs des éléments suivants :
- du charbon, de la lignite,
- des déchets municipaux,
- des déchets d'animaux,
- de la biomasse,
- des matières plastiques telles que le polyéthylène ou le polyéthylène téréphtalate etc.

Cette liste n'est pas limitative.

Dans l'exemple de réalisation de la figure 1, la matière carbonée est de la biomasse.

L'installation de la figure 1 comprend au moins les unités suivantes :
- une unité 10 de production d'un premier flux de gaz de synthèse à partir de la matière carbonée,
- une unité 12 de conditionnement du premier flux de gaz de synthèse ;
- une unité 14 d'ajout d'un flux d'hydrogène au premier flux de gaz de synthèse, pour former un second flux de gaz de synthèse ;
- une unité 16 de production d'un premier flux de produit à partir du second flux de gaz de synthèse ;
- éventuellement une première unité 18 de séparation, dans laquelle le premier flux de produit est séparé, par exemple, en un flux d'eau, en un second flux de produit et un premier flux de gaz destiné à être recyclé ;
- éventuellement une seconde unité 20 de séparation, dans laquelle le premier flux de gaz destiné à être recyclé est séparé en un flux de purge gazeuse et en des premiers et seconds flux recyclés ;
- une unité 22 d'électrolyse de l'eau ;
- éventuellement une unité 24 de séparation de l'air.

L'unité 10 de production du premier flux de gaz de synthèse comprend typiquement une unité 26 de gazéification de la biomasse produisant un flux intermédiaire, et une unité de reformage 30 produisant le premier flux de gaz de synthèse à partir du flux intermédiaire.

La biomasse peut éventuellement subir un prétraitement avant d'alimenter l'unité de gazéification 26. Ce prétraitement peut être une opération de broyage, de séchage, ou toute autre opération nécessaire pour mettre la biomasse dans un état adapté pour être traiter dans l'unité de gazéification.

L'unité de gazéification est choisie en fonction des caractéristiques de la biomasse à traiter. Elle est de type connu, elle ne sera pas décrite plus en détail ici. Elle est alimentée en biomasse via la ligne 34, en eau via la ligne 36, et en oxygène via la ligne 38. L'oxygène provient de l'unité d'électrolyse 22 et/ou de l'unité de séparation de l'air 24. Le flux de gaz intermédiaire quitte l'unité de gazéification par la ligne 28. Dans l'unité de gazéification 26, la biomasse s'est décomposée en un gaz comprenant essentiellement du méthane CH₄, de l'eau, du dioxyde de carbone, du monoxyde de carbone et de l'hydrogène.

Le flux intermédiaire passe ensuite dans une unité 40 d'ajustement du taux de vapeur d'eau dans le flux intermédiaire. Si le flux intermédiaire contient un excès de vapeur d'eau par rapport au taux nécessaire pour le bon fonctionnement de l'unité de reformage 30, une partie de la vapeur d'eau est séparée du flux intermédiaire et quitte l'unité 40 par la ligne 42. Le flux intermédiaire quitte l'unité 40 par la ligne 44, et alimente l'unité de reformage 30. Le flux d'eau quittant l'unité 40_par la ligne 42 peut alimenter l'unité de gazéification 26 ou l'unité d'électrolyse 22.

L'unité de reformage est typiquement une unité de reformage auto-thermale (ATR) ou une unité de reformage par oxydation partielle (POX : Partial Oxydation) ou une unité de reformage à la vapeur (SR). L'unité de reformage 30 est alimentée par le flux intermédiaire via la ligne 44, et est alimentée en oxygène à partir de l'unité d'électrolyse 22 ou de l'unité de séparation d'air 24, via la ligne 46. Elle reçoit également le premier flux de gaz recyclé, via la ligne 48. Elle est éventuellement alimentée en vapeur d'eau par la ligne 49, au cas où le flux intermédiaire ne comprend pas assez de vapeur d'eau pour le bon fonctionnement de l'unité de reformage 30. Le premier flux de gaz recyclé comprend essentiellement du CO, du CO₂, du CH₄ de l'H₂ et des hydrocarbures légers. Dans l'unité de reformage 30, les molécules de méthane provenant du flux intermédiaire et les molécules de méthane ainsi que les hydrocarbures légers provenant du premier flux de gaz recyclé sont décomposées et converties en CO, CO₂ et H₂.

L'unité de reformage produit le premier flux de gaz de synthèse, qui est dirigé vers l'unité de conditionnement des gaz 12 via la ligne 32.

Le premier flux de gaz de synthèse comprend majoritairement du monoxyde de carbone CO, du dioxyde de carbone CO₂, de l'hydrogène H₂ et de l'eau. Il comprend également d'autres gaz, en plus faible quantité. Ces autres gaz sont, entre autres, des hydrocarbures non convertis et des impuretés (H₂S, NOx, etc).

L'unité de conditionnement des gaz 12 reçoit le premier flux de gaz de synthèse venant de l'unité de reformage 30 et sépare le monoxyde de carbone et l'hydrogène du dioxyde de carbone, de la vapeur d'eau et des autres impuretés tels que H₂S par exemple. Dans l'unité de conditionnement des gaz 12, le premier flux de gaz de synthèse est ainsi séparé en un premier flux de gaz de synthèse purifié contenant essentiellement du CO et de l'hydrogène, et en un ou plusieurs flux de gaz séparés. Le premier flux de gaz purifié quitte l'unité 12 via la ligne 50, les flux de gaz séparés via les lignes 52 et 54.

Le premier flux de gaz de synthèse purifié est dirigé à partir de l'unité de conditionnement des gaz 12 jusqu'à l'unité 14 d'ajout d'hydrogène, via la ligne 50. Les flux de gaz séparés, avant d'être rejetés dans l'atmosphère, peuvent subir un traitement visant par exemple à récupérer la vapeur d'eau de manière à alimenter l'unité d'électrolyse 22 ou l'unité de gazéification 26. Le CO₂ peut être rejeté à l'atmosphère, ou valorisé ou stocké.

Dans l'unité 14, une quantité déterminée d'hydrogène est ajoutée au premier flux de gaz de synthèse purifié, de manière à former un second flux de gaz de synthèse. L'hydrogène provient de l'unité d'électrolyse 22, via la ligne 56. Dans l'unité 14, le second flux de gaz recyclé est également mélangé à l'hydrogène et au premier flux de gaz de synthèse purifié. Le second flux de gaz recyclé alimente l'unité 14 via la ligne 58. Ce second flux comprend principalement du H₂ et du CO, et des quantités plus faibles de CO₂, du méthane et des hydrocarbures légers. Le second flux de gaz de synthèse quitte l'unité 14 via la ligne 60, et alimente l'unité de production 16.

L'unité de production 16, dans l'exemple de réalisation de la figure 1, fonctionne selon le procédé de Fisher-Tropsch. Dans cette unité, le monoxyde de carbone CO réagit avec l'hydrogène pour produire des hydrocarbures. Cette réaction est catalysée par des catalyseurs adéquats. L'unité de production est de type connu et ne sera pas décrite plus en détails ici.

Le premier flux de produit quitte l'unité 16 via la ligne 62 et est dirigé jusqu'à la première unité de séparation 18. Le premier flux de produit comprend la fraction du CO et du H₂ issus du second flux de gaz de synthèse et n'ayant pas réagi dans l'unité 16. Il comprend également tous les produits issus des réactions au sein de l'unité 16, et en particulier un grand nombre d'hydrocarbures de natures différentes, ainsi que de l'eau. Il comprend encore, entre autres, du méthane CH₄, et des chaînes en C2, C3, C4,..., C100, etc. Les hydrocarbures comprennent entre autres des naphtas, des cires, des wax (cires) et des produits valorisables tels que par exemple du diesel, et/ou du kérosène, etc.

Dans la première unité de séparation, le premier flux de produit est séparé en au moins trois flux :
- un flux d'eau avec certains produits organiques dissous en faible proportion (alcools, acides organiques) ;
- un second flux de produit final comprenant la plus grande partie des produits condensables (pour le procédé Fischer-Tropsh de C4 à C100) ;
- un premier flux de gaz destiné à être recyclé, comprenant au moins le CO, le CO₂, le H₂, et des hydrocarbures légers, notamment le CH₄.

Le second flux de produit final quitte la première unité de séparation 18 via la ligne 64. Il peut être dirigé vers une unité de post-traitement, visant à séparer les différents composants de ce flux les uns des autres. L'unité de post-traitement peut également être une unité visant à convertir ces composants en un autre produit valorisable, par exemple du diesel, etc.

Le flux d'eau quitte la première unité de séparation 18 via la ligne 66. Il est par exemple dirigé vers l'unité d'électrolyse 22 ou vers l'unité de gazéification 26, éventuellement après traitement. Le premier flux de gaz destiné à être recyclé quitte l'unité 18 par la ligne 68 et est dirigé vers la seconde unité de séparation 20.

Dans cette seconde unité de séparation, ce flux est divisé en trois flux différents : le flux de purge (ligne 70), le flux de recyclage vers l'unité 14 via la ligne 58 et le flux de recyclage vers l'unité 30 via ligne 48. Le débit de chaque flux est choisi afin d'optimiser le rendement du procédé. Normalement la valeur du débit de purge est la plus faible des trois. Dans un exemple de réalisation, les trois flux présentent exactement la même composition pour tous les composés. Cependant, il peut être prévu une unité de séparation permettant d'enlever efficacement les impuretés des flux de recyclage vers les unités 14 et 30, et de les transférer dans le débit de purge.

Le flux de purge est rejeté dans l'atmosphère, après un éventuel post-traitement. Ce post-traitement peut constituer à brûler les hydrocarbures légers (valorisation énergétique), à séparer certains éléments gazeux dont le rejet à l'atmosphère est réglementé, etc.

L'unité d'électrolyse 22 permet de produire un flux d'hydrogène et un flux d'oxygène par électrolyse de l'eau. L'eau alimentant l'unité d'électrolyse 22 est issue d'une source d'eau extérieure au procédé. Afin de limiter la consommation total d'eau du procédé, une partie de cette alimentation d'eau peut être aussi issue de l'unité de conditionnement des gaz 12, et/ou de la première unité de séparation 18. L'hydrogène quitte l'unité d'électrolyse via la ligne 56. L'oxygène quitte l'unité d'électrolyse via la ligne 74, et alimente l'unité de gazéification ou l'unité de reformage. L'unité d'électrolyse 22 est également alimentée en électricité via la ligne électrique 76. La ligne électrique 76 est typiquement raccordée à un réseau de distribution électrique, le réseau n'étant pas dédié à l'unité d'électrolyse 22 et desservant d'autres consommateurs extérieurs à l'unité de production de méthanol ou d'hydrocarbures.

Dans l'unité de séparation d'air 24, l'oxygène de l'air est séparé des autres gaz, tel que l'azote. Dans certains exemples de réalisation, l'unité de séparation d'air 24 n'est pas nécessaire, l'unité d'électrolyse 22 fournissant suffisamment d'oxygène pour alimenter l'unité de production 10.

Les différentes unités de l'installation sont pilotées par une unité de pilotage 78 programmée pour mettre en oeuvre la méthode de production qui va être décrite plus bas.

L'installation de production de la figure 2 va maintenant être décrite. Seuls les points par lesquels cette installation diffère de celle de la figure 1 seront détaillés ci-dessous. Les éléments identiques ou assurant la même fonction dans les deux installations seront désignés par les mêmes références.

Dans cette installation, l'unité de reformage 30 est une unité de reformage à la vapeur (SR, Steam Reforming). L'unité de reformage 30 comprend une sous-unité de combustion 80 et une sous-unité de reformage 82. Le flux gaz intermédiaire après passage dans l'unité d'ajustement du taux de vapeur d'eau 40, alimente la sous-unité 80. Dans la sous-unité 80, ce gaz est chauffé de manière indirecte, par échange de chaleur avec un gaz de combustion. Le flux de gaz intermédiaire réchauffé quitte la sous-unité de combustion 80 par la ligne 84 et est dirigé vers la sous-unité de reformage 82. Le gaz de combustion, dans l'exemple de la figure 2, provient de la seconde unité de séparation 20. Il comporte typiquement du CO, du CO₂, du CH₄ et de l'H₂ et des hydrocarbures légers. Il est dirigé à partir de l'unité 20 jusqu'à une unité d'ajout 88, via la ligne 86. Dans l'unité d'ajout 88, un comburant, par exemple de l'air ou de l'oxygène, est ajouté au gaz de combustion, via la ligne 90. Le gaz de combustion est ensuite dirigé via la ligne 92 de l'unité 88 jusqu'à la sous-unité de combustion, dans laquelle il brûle en dégageant de la chaleur. Les gaz brûlés quittent la sous-unité 80 par la ligne 94 et constituent la purge. Ils sont rejetés à l'atmosphère, éventuellement après purification.

Dans la sous-unité de reformage 82, le méthane du flux de gaz intermédiaire réchauffé est décomposé et converti en CO, CO₂ et H₂ par les réactions de reformage.

Le premier flux de gaz de synthèse, quittant la sous-unité de reformage 82, est dirigé vers l'unité de conditionnement des gaz par la ligne 32.

Dans la méthode de production de l'invention, il est prévu de moduler la demande de puissance électrique de l'unité d'électrolyse 22, de manière à s'adapter à la puissance électrique disponible sur le réseau. Ceci permet en premier lieu de diminuer la puissance électrique de l'unité d'électrolyse en période de pointe, c'est-à-dire quand les autres consommateurs électriques raccordés au réseau appellent une puissance importante. En effet, la puissance électrique consommée varie au cours d'une même journée, avec par exemple un pic en fin de journée. En revanche, la consommation électrique pendant la nuit est réduite.

La consommation électrique varie également au long de l'année, cette consommation étant plus importante pendant les mois d'hiver du fait du grand nombre de consommateurs ayant des moyens de chauffage électrique, et est plus réduite en été, au moins dans les pays où la climatisation n'est pas généralisée.

Moduler la puissance électrique de l'unité de l'électrolyse est également particulièrement utile dans le cas où le réseau de distribution d'électricité est alimenté par des sources électriques de puissance variable, comme des sources éoliennes ou solaires. La puissance électrique fournie par de telles sources varie en effet en fonction des conditions météorologiques.

Ainsi, dans la méthode de production de l'invention, la puissance électrique consommée pour produire le flux d'hydrogène est plus importante à certaines périodes dites creuses, et moins importantes à d'autres périodes dites de pointe.

Ainsi, l'unité d'électrolyse 22 produit un flux d'hydrogène plus faible en période de pointe et plus important en période creuse.

Pour compenser ce manque en hydrogène et donc obtenir le bon rapport H₂/CO à l'entrée de l'unité 16 (typiquement de l'ordre de 2.1 pour une application Fischer-Tropsch), l'unité de pilotage 78 est programmée pour mettre en oeuvre la stratégie suivante.

Les conditions opératoires de l'unité de reformage 30 peuvent être modifiées afin de produire une quantité plus au moins importante d'hydrogène. Typiquement le rapport molaire H₂/CO obtenu en sortie de l'unité 30 peut varier entre 0.6 et 3. En effet, le fonctionnement de cette unité est déterminée par la température, la pression de réaction, et la composition des flux entrants dans l'unité de reformage 30, notamment le rapport H₂O/C et O₂/C (respectivement le débit molaire total d'eau entrant dans l'unité 30 sur le débit molaire total d'atomes de carbone contenus dans les composés organiques entrants dans l'unité 30, et le débit molaire total d'oxygène entrant dans l'unité 30 sur le débit molaire total d'atomes de carbone contenus dans les composés organiques entrants dans l'unité 30).

On entend par « débit molaire total d'une espèce entrant dans l'unité de reformage 30 » la somme des débits molaires de cette espèce dans tous les flux entrant dans l'unité 30. Dans l'exemple de la figure 1, le débit molaire total d'atomes de carbone contenus dans les composés organiques correspond à la somme des débits molaires d'atomes de carbone dans les différents composés organiques (hydrocarbures) dans le flux intermédiaire et dans le premier flux de gaz recyclé. Le débit molaire total d'eau H₂O correspond à la somme des débits molaires d'eau dans le flux de gaz intermédiaire 44, 49 et dans le premier flux de gaz recyclé (48). Le débit molaire total d'oxygène correspond essentiellement au débit d'oxygène apporté par la ligne 46, puisque le débit molaire d'oxygène dans les autres flux entrant dans l'unité de reformage 30 est pratiquement nul.

En pratique, quand le méthane CH₄ est majoritaire dans les composés organiques entrants dans l'unité de reformage 30, il est possible d'approximer les ratios ci-dessus pour des ratios H₂O / CH₄ et O₂/ CH₄. H₂O et O₂ ont la même signification que ci-dessus. CH₄ correspond au débit molaire total de méthane dans tous les flux entrant dans l'unité de reformage 30.

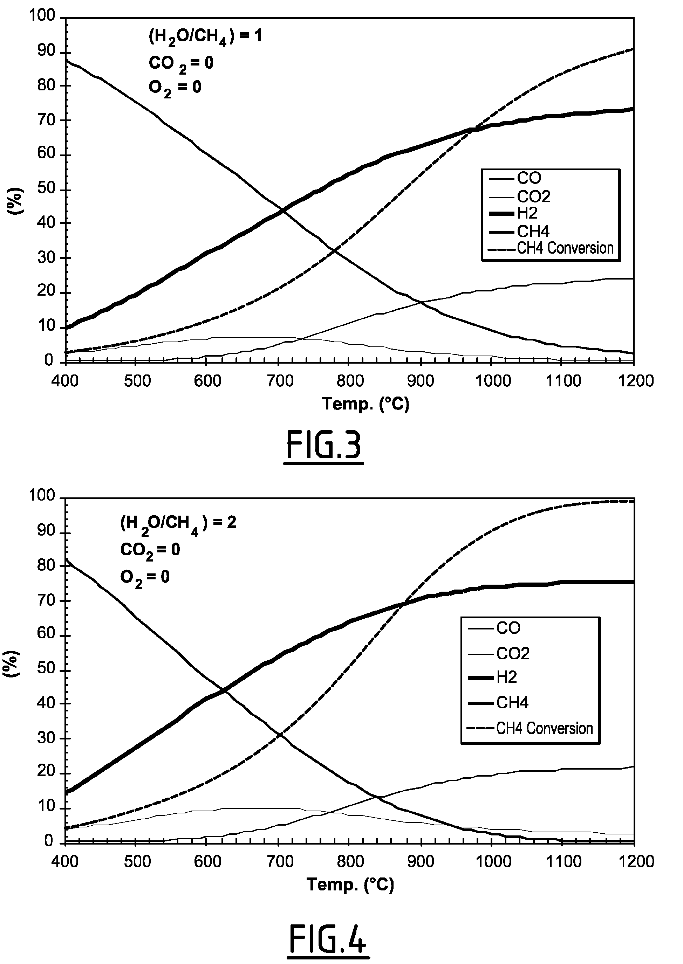

Les figures 3 à 10 montrent des calculs obtenus par simulation afin d'illustrer de façon simplifiée l'évolution des différentes fractions molaires des gaz produits dans une unité type ATR ou SR. Le réacteur considéré est isotherme et la pression de fonctionnement de 30 bars. Les concentrations ainsi obtenues sont à l'équilibre thermodynamique (réacteur Gibbs). En outre, il a été considéré que le réacteur était chauffé parfaitement à la température souhaitée.

Les figures 3 à 6 montrent l'évolution des fractions molaires en base sèche du CH₄, du H₂, du CO et du CO₂ à l'équilibre, et le taux de conversion de méthane, à la sortie de l'unité de reformage de la figure 1, en fonction de la température de réaction. La température est en abscisse, les fractions molaires et le taux de conversion du méthane sont en ordonnée. La température est exprimée en degrés C et les autres paramètres en %.

Pour une unité de reformage du type ATR comprend deux étapes, intégrées dans un seul réacteur :
- une étape de combustion ou d'oxydation partielle, à laquelle on injecte de l'oxygène afin d'augmenter la température des gaz entrants jusqu'aux conditions de réaction souhaitées, sous l'effet de réactions exothermiques,
- une deuxième étape endothermique, au cours de laquelle les réactions de reformage ont lieu dans un lit catalytique fixe.

Les températures dans le lit catalytique varient typiquement entre 800 et 1000°C, sous une pression de 20 à 100 bars. Dans le haut du lit catalytique, les températures peuvent atteindre 1100 à 1400 °C. Le catalyseur est par exemple à base de nickel sur un support en spinelles. La température à l'étape de combustion est de l'ordre de 2000°C.

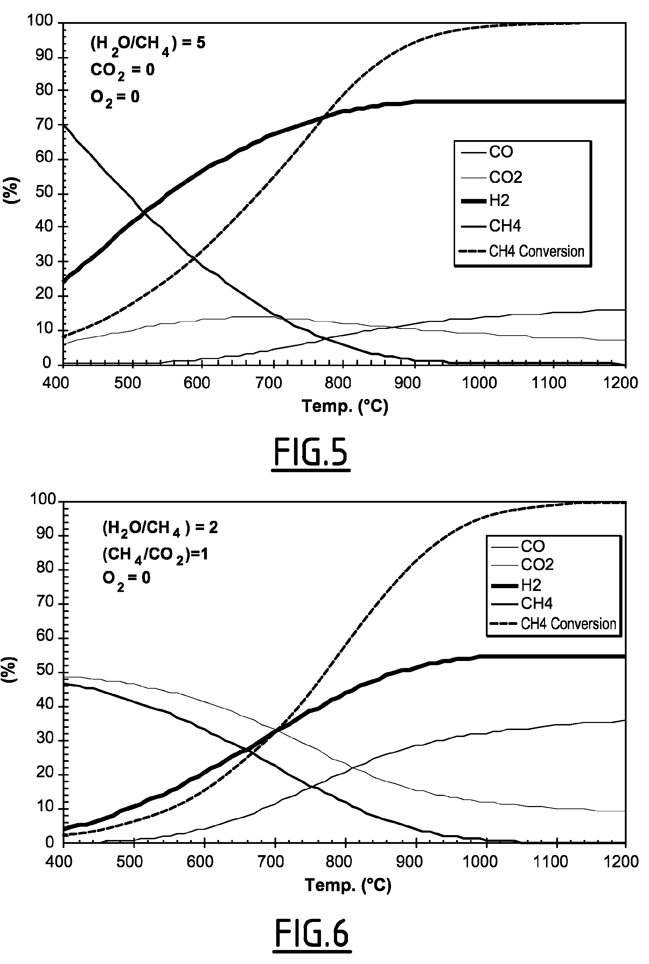

Dans les figures 3, 4, et 5, le flux de gaz entrant ne comporte pas de CO₂. En revanche, le rapport H₂O total sur CH₄ total à l'entrée du réacteur est de 1 sur la figure 3, 2 sur la figure 4, et 5 sur la figure 5. Sur la figure 6, le rapport H₂O total sur CH₄ total dans les gaz entrants est de 2, et le rapport CH₄ total sur CO₂ total dans les gaz entrants est de 1.

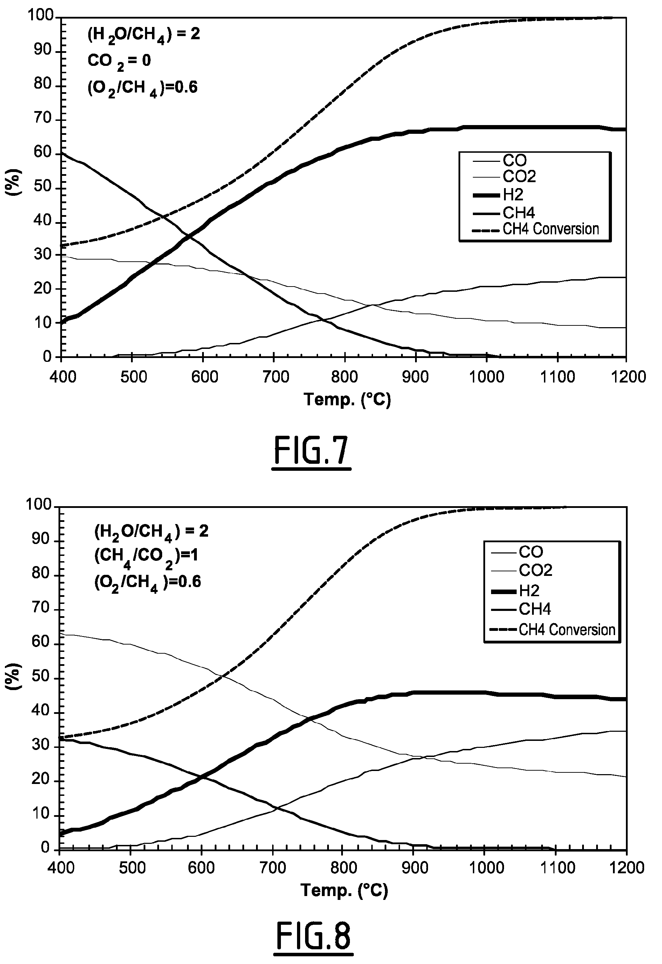

Les figures 7 et 8 donnent les mêmes grandeurs en fonction de la température que les figures 3 à 6, pour une unité de reformage à la vapeur telle que celle représentée sur la figure 2. Sur les figures 7 et 8, le rapport H₂O total sur CH₄ total à l'entrée de l'unité de reformage est égal à 2. De même, le rapport O₂ total sur CH₄ total à l'entrée de l'unité de reformage vaut 0,6 dans les deux cas. En revanche, pour la figure 7, il n'y a pas de CO₂ dans les gaz entrants dans l'unité de reformage. Pour la figure 8, le rapport CH₄ total sur CO₂ total à l'entrée de l'unité de reformage vaut 1.

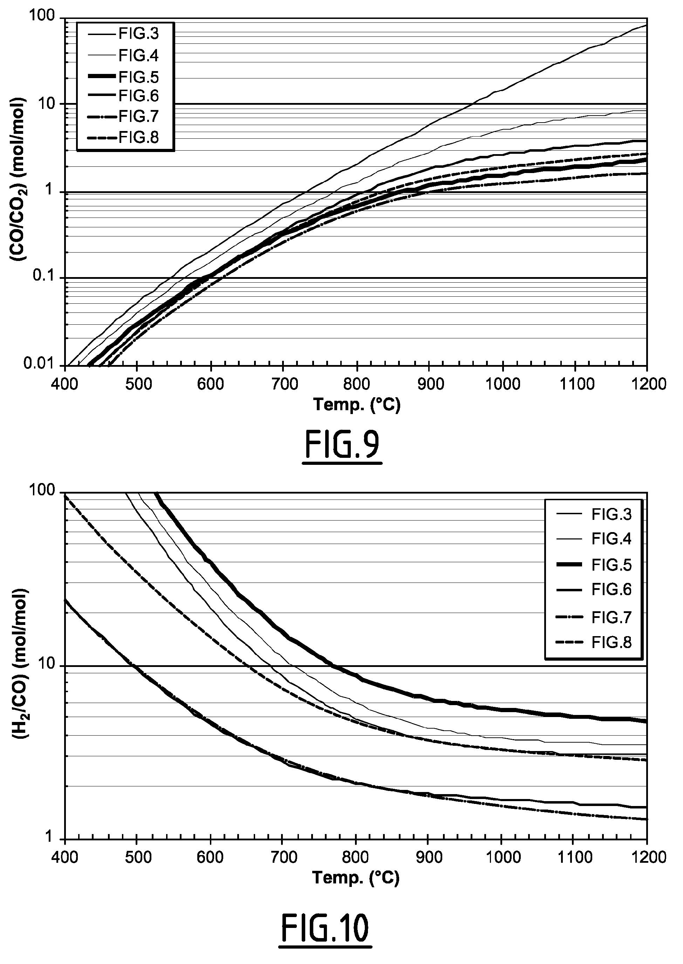

Les figures 9 et 10 montrent l'évolution des rapports respectivement CO/CO₂ et H₂/CO à l'équilibre, c'est-à-dire en sortie du réacteur, en fonction de la température de réaction pour les six cas correspondants aux figures 3 à 8.

Il ressort clairement que :
a) Le taux de conversion du méthane est très sensible à la température de réaction et la conversion du méthane est favorisée par des températures élevées,
b) Un rapport (H₂O/CH₄) important favorise la conversion de méthane et donc la production de gaz de synthèse (figures 3, 4 et 5),
c) La présence de CO₂ et de O₂ à l'entrée du réacteur fait diminuer le rapport (H₂/CO) ainsi que leurs quantités relatives obtenues (figures 7 et 8),
d) La teneur en CO₂ croît avec l'augmentation du rapport (H₂O/CH₄) à l'entrée du réacteur (figures 3, 4 et 5),
e) Sur les figures 9 et 10, on peut constater que la présence de O₂ à l'entrée du reformeur fait diminuer de manière importante le rapport H₂/CO.
f) Le rapport H₂/CO décroît avec la température de réaction,
g) Dans la totalité des figures, on peut constater qu'il existe un compromis entre la conversion de méthane et le rapport H₂/CO obtenu.

La conversion du méthane est favorisée pour des pressions plus faibles. Toutefois, cette solution pour faire varier le rapport H₂/CO n'est pas privilégiée, car une diminution importante de la pression de fonctionnement impliquerait d'ajouter une unité de compression en aval afin d'obtenir une pression adaptée pour l'unité 16 (typiquement entre 25 et 35 bar pour une unité Fischer-Tropsch).

En outre, bien que la température de réaction soit un paramètre très influent sur le rapport H₂/CO, il faut faire varier ce paramètre avec prudence, car la cinétique de réaction, qui dépend de l'activité du catalyseur, et les vitesses de réaction, sont en conséquence modifiées..

Ainsi, pour compenser une baisse de la puissance électrique consommée par l'unité d'électrolyse 22, et donc une baisse de la quantité d'hydrogène produit par cette unité 22, il convient de réduire la température de fonctionnement de l'unité de reformage 30, et/ou de diminuer le rapport O₂/C à l'entrée de l'unité de reformage 30 et/ou d'augmenter le rapport H₂O/C à l'entrée de l'unité de reformage.

Dans une variante moins privilégiée, il convient de diminuer la pression de fonctionnement de l'unité de reformage 30.

Dans la pratique, il n'est pas possible d'effectuer simultanément toutes ces modifications, ou seulement dans des plages restreintes. En effet, une diminution de la température affecte les cinétiques de réaction et il est possible que les conditions d'équilibre à la sortie du réacteur ne soient pas atteintes pour des températures trop faibles. Par ailleurs, la quantité d'oxygène détermine la température de réaction du reformeur et une augmentation de rapport H₂O/C implique une demande d'oxygène, car il y a une proportion plus importante de réactions de reformage (réactions endothermiques). Ainsi, il est nécessaire de déterminer au cas par cas, en fonction de la composition du gaz intermédiaire et du type d'unité de reformage utilisé, sur quels paramètres jouer et dans quelle mesure.

La figure 10 montre que, pour les cas de calcul considérés sur les figures 6 à 8, augmenter le ratio H₂O total sur CH₄ total en entrée de l'unité de reformage permettait d'augmenter le ratio H₂/CO en sortie de l'unité de reformage. L'unité de pilotage 78 est programmée pour faire varier le rapport H₂O/C (ou H₂O/CH₄) total à l'entrée de l'unité de reformage au moins d'un facteur compris entre 1,1 et 5, de préférence compris entre 1,5 et 3.

Toutefois, dans certains autres exemples de réalisation, par exemple avec d'autres types de matière carbonée et d'autres conditions de fonctionnement de l'unité de reformage, le rapport H₂O total / CH₄ total à l'entrée de l'unité de reformage devra être diminué par l'unité de pilotage 78 pour augmenter le rapport H₂/CO en sortie de l'unité de reformage.

Selon les cas, il sera nécessaire d'augmenter ou de baisser le rapport O₂/CH₄ à l'entrée de l'unité de reformage pour augmenter le rapport H₂O/CO à la sortie de cette même unité de reformage.

Dans l'exemple montré ci-dessous [Tableau 1], le seul paramètre de pilotage du rapport H₂/CO à la sortie de l'unité 30 est le rapport H₂O/C. La valeur du rapport O₂/C est ajustée de façon à avoir une température de reformage constante.

Le tableau 1 montre les performances des procédés de la figure 1 et de la figure 2, en période de pointe et en période creuse quand l'unité 16 est un réacteur Fischer-Tropsch. Dans les quatre cas de calcul, le ratio H₂/CO à l'entrée de l'unité 16 est de 2,1. En période creuse, la puissance électrique consommée par l'unité d'électrolyse est importante (de l'ordre de 150 MW). En période de pointe, la puissance électrique consommée par l'unité d'électrolyse est réduite aux environs de 80 MW, mais le ratio H₂O/CH₄ à l'entrée de l'unité de reformage est augmenté. Ce ratio vaut 1,15 en période creuse, et vaut 5 en période de pointe. On constate une diminution de la puissance nette demandée par l'électrolyseur de l'ordre de 45%. Cependant, inévitablement les produits FT diminuent de l'ordre de 17%. En effet, le rendement en carbone diminue parce qu'une partie du CO est convertie en CO₂ et H₂ afin de combler le manque en hydrogène électrolytique. De même, la quantité de CO₂ rejetée du système augmente. Ce CO₂ peut être stocké pour être utilisé dans un autre type de valorisation ou faire la séquestration.

La composition du gaz intermédiaire issu de l'unité de gazéification est indiquée dans le tableau 2, de même que le débit massique du flux de gaz intermédiaire, la température et la pression sortie de l'unité de gazéification.

Le tableau met en évidence que le ratio H₂/CO en sortie de l'unité de reformage peut varier de 0,7 en période creuse à 1,2 en période de pointe, pour le cas de calcul considéré. Le débit de produit à la sortie de l'unité de Fischer-Tropsch 16 est de l'ordre de 3,4 kg/s en période creuse, et 2,8 en période de pointe.

La méthode de l'invention, et l'installation de production correspondante, présentent de multiples avantages.

Elle permet d'adapter la puissance appelée par l'électrolyseur aux disponibilités du réseau de distribution électrique, ce qui contribue à stabiliser le fonctionnement du réseau de distribution au moins au niveau local. Par ailleurs, ceci permet de réduire la consommation électrique de l'électrolyseur au moment où les prix de l'électricité sont les plus élevés, pour augmenter la consommation électrique au moment où les prix de l'électricité sont plus faibles. La rentabilité de l'installation en est augmentée. Le fait de pouvoir moduler la puissance électrique consommée par l'unité d'électrolyse permet de faire fonctionner celui-ci avec de l'électricité provenant non pas d'un réseau de distribution électrique local, mais directement à partir d'une source électrique renouvelable, par exemple des éoliennes.

Il est possible d'adapter le fonctionnement de l'installation sans recourir à un stockage massif d'hydrogène ou de puissance électrique.

La puissance électrique consommée par l'électrolyseur peut être fortement réduite, la quantité de l'hydrocarbure produite étant réduite de manière modérée. La quantité d'hydrocarbure produite par MW consommé est nettement plus élevée en période de pointe que en période creuse, comme le montre le tableau 1. Dans les exemples de ce tableau, une réduction de plus de 40% de la puissance électrique consommée par l'unité d'électrolyse entraîne une réduction de moins de 20% de la quantité d'hydrocarbures produite.

Par rapport à ces résultats, si on prend en compte aussi la flexibilité du procédé vis-à-vis du débit d'alimentation du réacteur de Fischer-Tropsch, il est possible d'arriver à une réduction de plus de 50% de la puissance électrique consommée par l'unité d'électrolyse, pour une réduction de moins de 30% de la quantité d'hydrocarbures produites.

La méthode et l'installation de production peuvent présenter de multiples variantes.

L'unité de production 16 peut ne pas fonctionner selon le procédé de Fischer-Tropsch, mais être une unité de production de méthanol. Le méthanol peut être le produit final, ou être soumis à un post-traitement pour être converti en hydrocarbure, par exemple selon le procédé MTG (Méthanol To Gazoline).

L'unité de séparation de l'air 24 n'est pas nécessaire dans tous les cas, l'unité d'électrolyse étant dans certains cas suffisante pour fournir la quantité d'oxygène nécessaire à l'unité de gazéification et à l'unité de reformage.

**Tableau 1 : Comparaison des performances du procédé FT avec une unité ATR et SR**

| | **Figure 2** **(SR) Période creuse** | **Figure 1** **(ATR) Période creuse** | **Figure 2** **(SR) Période de pointe** | **Figure 1** **(ATR) Période de pointe** |
|---|---|---|---|---|
| **Variables d'étude** | | | | |
| (H₂/CO) (mol) Ligne 60 | 2.1 | 2.1 | 2.1 | 2.1 |
| (H₂O/CH₄) (mol) Entrée réformeur Ligne 44 | 1.15 | 1.15 | 5 | 5 |

| **ATR ou SR (unité 30)** | | | | |
|---|---|---|---|---|
| (H₂/CO) (mol) sortie réformeur Ligne 32 | 0.69 | 0.73 | 1.20 | 1.19 |
| Alimentation O₂ réformeur (kg/s) Ligne 46 | --- | 3.392 | --- | 2.478 |

| **Combustion** | | | | |
|---|---|---|---|---|
| Alimentation O₂ (kg/s) Ligne 90 | 3.697 | --- | 2.522 | --- |

| **Electrolyseur (unité 22)** | | | | |
|---|---|---|---|---|
| H₂ (kg/s) Ligne 56 | 0.880 | 0.846 | 0.476 | 0.4682 |
| O₂ (kg/s) Ligne 74 | 6.987 | 6.714 | 3.778 | 3.716 |
| MW consommé Ligne 76 | **152** | **146** | **82** | **81** |

| **Procédé FT (unité 16)** | | | | |
|---|---|---|---|---|
| Conversion (%) | 70 | 70 | 70 | 70 |
| Alimentation gaz de synthèse (CO+H₂) (kg/s) Ligne 50 | 9.11 | 9.00 | 7.91 | 7.73 |
| Alimentation procédé FT (kg/s) Ligne 50 | 12.43 | 12.37 | 11.39 | 11.29 |
| Alimentation réacteur FT (kg/s) Ligne 60 | 32.33 | 32.40 | 30.52 | 30.63 |
| Produits (kg/s) Ligne 64 | **3.402** | **3.354** | **2.847** | **2.781** |
| | | | | |
| Ratio purge (%) | 19 | 5 | 17 | 5 |
| Purge Gaz (kg/s) Ligne 70/94 | 4.517 | 1.199 | 3.693 | 1.180 |
| CO₂ rejeté (kg/s) Ligne 52 | **1.945** | **6.859** | **4.757** | **8.679** |
| | | | | |
| (Kg Produits) / (MWh électrolyseur) | 80.5 | 82.6 | 125.0 | 123.6 |

| | | | | |
|---|---|---|---|---|
| * Les unités ATR et SR fonctionnent à 950°C et 28.5 bar. | | | | |

**Tableau 2 : Composition base sèche du gaz intermédiaire à la sortie de l'unité de gazéification**

| | | |
|---|---|---|
| CO | 19.2 | % mol |
| CO₂ | 45.0 | % mol |
| H₂ | 19.0 | % mol |
| CH₄ | 13.2 | % mol |
| Autres composés organiques | 3.5 | % mol |
| H₂/CO | 1.00 | mol/mol |
| Pression | 29 | bar |
| Température | 950 | °C |
| Débit massique | 20.4 | kg/s |
| Ethane | 0.08 | % v/v |
| Ethylène | 1.53 | % v/v |
| Méthane | 8.17 | % v/v |
| CO | 11.86 | % v/v |
| CO₂ | 27.92 | % v/v |
| H₂ | 11.79 | % v/v |
| H₂O | 37.69 | % v/v |
| N₂ | 0.06 | % v/v |
| Ar | 0.06 | % v/v |
| Ammoniac | 0.29 | % v/v |
| H₂S | 0.01 | % v/v |
| BTX - Benzène | 0.26 | % v/v |
| Tars-Naphtalène | 0.27 | % v/v |
| H₂/CO | 1.00 | mol/mol |
| H₂O/CH₄ | 4.6 | mol/mol |

## Revendications

1. Méthode de production de méthanol et/ou d'hydrocarbures à partir d'au moins une matière carbonée, la méthode comprenant les étapes suivantes :
- produire un gaz de synthèse à partir de la matière carbonée, selon un procédé comprenant au moins une opération de reformage d'un flux de gaz intermédiaire issu de la matière carbonée, le flux de gaz de synthèse comprenant au moins de l'hydrogène et du monoxyde de carbone, le gaz de synthèse ayant un premier ratio molaire hydrogène/ monoxyde de carbone dans des premières conditions opératoires pour l'opération de reformage ;
- produire un flux d'hydrogène à partir d'une matière première hydrogénée et d'une première puissance électrique consommée, le flux d'hydrogène ayant un premier débit molaire pour ladite première puissance électrique consommée ;
- produire le méthanol et/ou les hydrocarbures à partir du gaz de synthèse et du flux d'hydrogène ;
- baisser la puissance électrique consommée pour produire le flux d'hydrogène jusqu'à une seconde puissance électrique inférieure à la première puissance électrique, le flux d'hydrogène ayant un second débit molaire inférieur au premier débit molaire pour la seconde puissance électrique consommée, et se placer dans des secondes conditions opératoires différentes des premières pour l'opération de reformage pour compenser la baisse du débit molaire du flux d'hydrogène, le gaz de synthèse ayant un second ratio molaire hydrogène/ monoxyde de carbone supérieur au premier dans les secondes conditions opératoires.

2. Méthode selon la revendication 1, **caractérisée en ce que** dans les premières conditions opératoires, l'opération de reformage est effectuée à une première température, dans les secondes conditions opératoires, l'opération de reformage est effectuée à une seconde température inférieure à la première température.

3. Méthode selon la revendication 1 ou 2, **caractérisée en ce que** dans les premières conditions opératoires, l'opération de reformage est effectuée à une première pression, dans les secondes conditions opératoires, l'opération de reformage est effectuée à une seconde pression inférieure à la première pression.

4. Méthode selon l'une quelconque des revendications précédentes, **caractérisée en ce que** l'opération de reformage est effectuée dans une unité de reformage recevant en entrée une pluralité de flux d'entrée contenant des molécules organiques, dont le flux intermédiaire, l'unité de reformage recevant des différents flux d'entrée au total un débit molaire total d'atomes de carbone contenus dans les molécules organiques et un débit molaire total d'eau H₂O, l'opération de reformage passant des premières conditions opératoires aux secondes conditions opératoires par modification d'un ratio du débit molaire total d'eau H₂O sur le débit molaire total d'atomes de carbone contenus dans les molécules organiques.

5. Méthode selon la revendication 4, **caractérisée en ce que** le ratio du débit molaire total d'eau H₂O sur le débit molaire total d'atomes de carbone contenus dans les molécules organiques est égal à une première valeur dans les premières conditions opératoires, et est égal à une seconde valeur supérieure à la première dans les secondes conditions opératoires.

6. Méthode selon la revendication 5, **caractérisée en ce que** la seconde valeur est comprise entre 1,1 et 5 fois la première valeur, de préférence entre 1,5 et 3 fois la première valeur.

7. Méthode selon l'une quelconque des revendications précédentes, **caractérisée en ce que** l'opération de reformage est effectuée dans une unité de reformage recevant en entrée une pluralité de flux d'entrée, dont le flux intermédiaire, l'unité de reformage recevant des différents flux d'entrée au total un débit molaire total d'atomes de carbone contenus dans les molécules organiques et un débit molaire total d'oxygène O₂, l'opération de reformage passant des premières conditions opératoires aux secondes conditions opératoires par modification d'un ratio du débit molaire total d'oxygène sur le débit molaire total d'atomes de carbone contenus dans le molécules organiques.

8. Méthode selon l'une quelconque des revendications précédentes, **caractérisée en ce que** le flux d'hydrogène est produit à partir d'électricité fournie par un réseau de distribution électrique desservant d'autres consommateurs.

9. Méthode selon l'une quelconque des revendications précédentes, **caractérisée en ce que** le flux intermédiaire est obtenu par gazéification de la matière carboné.

10. Installation de production de méthanol et/ou d'hydrocarbures à partir d'au moins une matière carbonée, l'installation comprenant :
- une unité (10) de production d'un gaz de synthèse à partir de la matière carbonée, comprenant au moins une unité de reformage (30) d'un flux de gaz intermédiaire issu de la matière carbonée, le flux de gaz de synthèse comprenant au moins de l'hydrogène et du monoxyde de carbone, le gaz de synthèse ayant un premier ratio molaire hydrogène/ monoxyde de carbone dans des premières conditions opératoires pour l'unité de reformage (30);
- une unité (22) de production d'un flux d'hydrogène à partir d'une matière première hydrogénée et d'une première puissance électrique consommée, le flux d'hydrogène ayant un premier débit molaire pour ladite première puissance électrique consommée ;
- une unité (16) de production de méthanol et/ou d'hydrocarbures à partir du gaz de synthèse et du flux d'hydrogène ;
- une unité (78) de pilotage programmée pour baisser la puissance électrique consommée pour produire le flux d'hydrogène jusqu'à une seconde puissance électrique inférieure à la première puissance électrique, le flux d'hydrogène ayant un second débit molaire inférieur au premier débit molaire pour la seconde puissance électrique consommée, et pour placer l'unité de reformage (30) dans des secondes conditions opératoires différentes des premières pour compenser la baisse du débit molaire du flux d'hydrogène, le gaz de synthèse ayant un second ratio molaire hydrogène/ monoxyde de carbone supérieur au premier dans les secondes conditions opératoires.

## Patentansprüche

1. Verfahren zur Herstellung von Methanol und/oder Kohlenwasserstoffen aus mindestens einem kohlenstoffhaltigen Material, wobei das Verfahren die folgenden Schritte umfasst:
- Erzeugen eines Synthesegases aus dem kohlenstoffhaltigen Material, gemäß eines Verfahrens umfassend mindestens eine Durchführung der Reformierung eines Zwischengasstroms, der von dem kohlenstoffhaltigen Material stammt, wobei der Strom des Synthesegases mindestens Wasserstoff und Kohlenstoffmonoxid umfasst, wobei das Synthesegas unter ersten Operationsbedingungen für die Durchführung der Reformierung ein erstes Molverhältnis von Wasserstoff/Kohlenstoffmonoxid hat;
- Erzeugen eines Wasserstoffstroms aus einem ersten hydrierten Material und einer ersten verbrauchten elektrischen Leistung, wobei der Wasserstoffstrom einen ersten molaren Gehalt für die erste verbrauchte elektrische Leistung hat;
- Erzeugen des Methanols und/oder der Kohlenwasserstoffe aus dem Synthesegas und dem Wasserstoffstrom;
- Senken der für das Erzeugen des Wasserstoffstroms verbrauchten elektrischen Leistung bis auf eine zweite elektrische Leistung, die geringer ist als die erste elektrische Leistung, wobei der Wasserstoffstrom für die zweite verbrauchte elektrische Leistung einen zweiten molaren Gehalt hat, der geringer ist als der erste molare Gehalt, und Einstellen von zweiten Operationsbedingungen, die verschieden sind von den ersten zur Durchführung der Reformierung, um das Herabsenken des molaren Gehalts des Wasserstoffstroms zu kompensieren, wobei das Synthesegas unter den zweiten Operationsbedingungen ein zweites Molverhältnis von Wasserstoff/Kohlenstoffmonoxid hat, das höher ist als das erste.

2. Verfahren gemäß Anspruch 1, **dadurch gekennzeichnet, dass**, unter den ersten Operationsbedingungen, die Durchführung der Reformierung bei einer ersten Temperatur erfolgt, und, unter den zweiten Operationsbedingungen, die Durchführung der Reformierung bei einer zweiten Temperatur erfolgt, die niedriger ist als die erste Temperatur.

3. Verfahren gemäß Anspruch 1 oder 2, **dadurch gekennzeichnet, dass**, unter den ersten Operationsbedingungen, die Durchführung der Reformierung bei einem ersten Druck erfolgt, und, unter den zweiten Operationsbedingungen, die Durchführung der Reformierung bei einem zweiten Druck erfolgt, der geringer ist als der erste Druck.

4. Verfahren gemäß einem der voranstehenden Ansprüche, **dadurch gekennzeichnet, dass** die Durchführung der Reformierung in einem Reformierungsaggregat erfolgt, das im Eintrittsbereich eine Vielzahl von Eintrittsströmen enthaltend organische Moleküle empfängt, darunter der Zwischenstrom, wobei das Reformierungsaggregat von den verschiedenen Eintrittsströmen insgesamt einen molaren Gesamtgehalt von Kohlenstoffatomen, enthalten in den organischen Molekülen, und einen molaren Gesamtgehalt von Wasser H₂O empfängt, wobei die Durchführung der Reformierung durch Modifizierung eines Verhältnisses des molaren Gesamtgehalts von Wasser H₂O zu dem molaren Gesamtgehalt von Kohlenstoffatomen, enthalten in den organischen Molekülen, von den ersten Operationsbedingungen in die zweiten Operationsbedingungen übergeht.

5. Verfahren gemäß Anspruch 4, **dadurch gekennzeichnet, dass** das Verhältnis des molaren Gesamtgehalts von Wasser H₂O zu dem molaren Gesamtgehalt der Kohlenstoffatome, enthalten in den organischen Molekülen, unter den ersten Operationsbedingungen gleich einem ersten Wert ist und unter den zweiten Operationsbedingungen gleich einem zweiten Wert ist, der höher ist als der erste.

6. Verfahren gemäß Anspruch 5, **dadurch gekennzeichnet, dass** der zweite Wert zwischen 1,1 und 5 mal des ersten Wertes ist, vorzugsweise zwischen 1,5 und 3 mal des ersten Wertes.

7. Verfahren gemäß einem der voranstehenden Ansprüche, **dadurch gekennzeichnet, dass** die Durchführung der Reformierung in einem Reformierungsaggregat erfolgt, das im Eintrittsbereich eine Vielzahl von Eintrittsströmen empfängt, darunter der Zwischenstrom, wobei das Reformierungsaggregat von den verschiedenen Eintrittsströmen einen molaren Gesamtgehalt von Kohlenstoffatomen, enthalten in den organischen Molekülen, und einen molaren Gesamtgehalt von Sauerstoff O₂ empfängt, wobei die Durchführung der Reformierung durch Modifizierung eines Verhältnisses des molaren Gesamtgehalts von Sauerstoff O₂ zu dem molaren Gesamtgehalt von Kohlenstoffatomen, enthalten in den organischen Molekülen, von den ersten Operationsbedingungen in die zweiten Operationsbedingungen übergeht.

8. Verfahren gemäß einem der voranstehenden Ansprüche, **dadurch gekennzeichnet, dass** der Wasserstoffstrom mit Elektrizität erzeugt wird, die von einem elektrischen Verteilernetzwerk geliefert wird, das andere Verbraucher bedient.

9. Verfahren gemäß einem der voranstehenden Ansprüche, **dadurch gekennzeichnet, dass** der Zwischenstrom durch Vergasung des kohlenstoffhaltigen Materials erhalten wird.

10. Anlage zur Herstellung von Methanol und/oder Kohlenwasserstoffen aus mindestens einem kohlenstoffhaltigen Material, wobei die Anlage umfasst:
- ein Aggregat (10) zur Erzeugung eines Synthesegases aus dem kohlenstoffhaltigen Material, umfassend mindestens ein Aggregat (30) zur Reformierung eines Zwischengasstroms, der von dem kohlenstoffhaltigen Material stammt, wobei der Synthesegasstrom mindestens Wasserstoff und Kohlenstoffmonoxid umfasst, wobei das Synthesegas unter den ersten Operationsbedingungen für das Reformierungsaggregat (30) ein erstes Molverhältnis von Wasserstoff/Kohlenstoffmonoxid hat;
- ein Aggregat (22) zur Erzeugung eines Wasserstoffstroms aus einem ersten hydrierten Material und einer ersten verbrauchten elektrischen Leistung, wobei der Wasserstoffstrom einen ersten molaren Gehalt für die erste verbrauchte elektrische Leistung hat;
- ein Aggregat (16) zur Erzeugung von Methanol und/oder Kohlenwasserstoffen aus dem Synthesegas und dem Wasserstoffstrom;
- ein Steuerungsaggregat (78), programmiert für das Senken der elektrischen Leistung, die für die Erzeugung des Wasserstoffstroms verbraucht wird, bis auf eine zweite elektrische Leistung, die geringer ist als die erste elektrische Leistung, wobei der Wasserstoffstrom für die zweite verbrauchte elektrische Leistung einen zweiten molaren Gehalt hat, der geringer ist als der erste molare Gehalt, und für das Einstellen der zweiten Operationsbedingungen für das Reformierungsaggregat (30), die verschieden sind von den ersten, um für das Herabsenken des molaren Gehalts des Wasserstoffstroms zu kompensieren, wobei das Synthesegas unter den zweiten Operationsbedingungen ein zweites Molverhältnis von Wasserstoff/Kohlenstoffmonoxid hat, das höher ist als das erste.

## Claims

1. A method for producing methanol and/or hydrocarbons from at least one carbonaceous material, the method comprising the following steps:
- producing a synthesis gas from the carbonaceous materials, according to a process comprising at least one operation for reforming an intermediate gas stream from the carbonaceous material, the stream of synthesis gas comprising at least hydrogen and carbon monoxide, the synthesis gas having a first hydrogen/carbon monoxide molar ratio under first operating conditions for performing the reforming;
- producing a hydrogen stream from a hydrogenated raw material and from a first consumed electric power, the hydrogen stream having a first molar flow rate for said first consumed electric power;
- producing methanol and/or hydrocarbons from the synthesis gas and from the hydrogen stream;
- decreasing the consumed electric power for producing the hydrogen stream, down to a second electric power below the first electric power, the hydrogen stream having a second molar flow rate below the first molar flow rate for the second consumed electric power, and setting second operating conditions different from the first for the reforming operation in order to compensate for the decrease of the molar flow rate of the hydrogen stream, the synthesis gas having a second hydrogen/carbon monoxide molar ratio greater than the first under the second operating conditions.

2. The method according to claim 1, **characterized in that**, under the first operating conditions, the reforming operation is carried out at a first temperature; under the second operating conditions, the reforming operation is carried out at a temperature below the first temperature.

3. The method according to claim 1 or 2, **characterized in that**, under the first operating conditions, the reforming operation is carried out at a first pressure; under the second operating conditions, the reforming operation is carried out at a second pressure below the first pressure.

4. The method according to any of the preceding claims, **characterized in that** the reforming operation is carried out in a reforming unit receiving at the inlet a plurality of inflows containing organic molecules, including the intermediate stream, the reforming unit receiving from the different inflows, all in all, a total molar flow rate of carbon atoms contained in the organic molecules and a total water H₂O molar flow rate, the reforming operation passing from the first operating conditions to the second operating conditions by modifying a ratio of the total water H₂O molar flow rate over the total molar flow rate of carbon atoms contained in the organic molecules.

5. The method according to claim 4, **characterized in that** the ratio of the total water H₂O molar flow rate over the total molar flow rate of carbon atoms contained in the organic molecules is equal to a first value under the first operating conditions, and is equal to a second value greater than the first under the second operating conditions.

6. The method according to claim 5, **characterized in that** the second value is comprised between 1.1 and 5 times the first value, preferably between 1.5 and 3 times the first value.

7. The method according to any of the preceding claims, **characterized in that** the reforming operation is carried out in a reforming unit receiving at the inlet a plurality of inflows, including the intermediate stream, the reforming unit receiving from the different inflows, all in all, a total molar flow rate of carbon atoms contained in the organic molecules and a total oxygen O₂ molar flow rate, the reforming operation passing from the first operating conditions to the second operating conditions by modifying a ratio of the total molar flow rate of oxygen over the total molar flow rate of carbon atoms contained in the organic molecules.

8. The method according to any of the preceding claims, **characterized in that** the hydrogen stream is produced from electricity provided by an electric distribution network serving other consumers.

9. The method according to any of the preceding claims, **characterized in that** the intermediate stream is obtained by gasification of the carbonaceous material.

10. A facility for producing methanol and/or hydrocarbons from at least one carbonaceous material, the facility comprising:
- a unit (10) for producing synthesis gas from the carbonaceous material, comprising at least one unit (30) for reforming an intermediate gas stream from the carbonaceous mater, the stream from synthesis gas comprising at least hydrogen and carbon monoxide, the synthesis gas having a first hydrogen/carbon monoxide molar ratio under the first operating conditions for the reforming unit (30);
- a unit (22) for producing a hydrogen stream from a hydrogenated raw material and from a first consumed electric power, the hydrogen stream having a first molar flow rate for said first consumed electric power;
- a unit (16) for producing methanol and/or hydrocarbons from the synthesis gas and from the hydrogen stream;
- a programmed control unit (78) for decreasing the consumed electric power for producing the hydrogen stream, down to a second electric power below the first electric power, the hydrogen stream having a second molar flow rate below the first molar flow rate for the second consumed electric power, and for setting the reforming unit (30) under second operating conditions different from the first for compensating for the decrease in the molar flow rate of the hydrogen stream, the synthesis gas having a second hydrogen/carbon monoxide molar ratio greater than the first under the second operating conditions.
